# NEUE EUROPÄISCHE PATENTSCHRIFT

(11) **EP 1 219 266 B2**
(45) Veröffentlichungstag und Bekanntmachung des Hinweises auf die Entscheidung über den Einspruch: **11.01.2012**
(45) Hinweis auf die Patenterteilung: 27.09.2006
(21) Anmeldenummer: 01126995.8
(22) Anmeldetag: 14.11.2001
(51) Int. Cl.: A61F 2/30, A61F 2/44, A61F 2/46

(54) **Implantat zum Einsetzen zwischen Wirbelkörper sowie Operationsinstrument zur Handhabung des Implantats**
Implant for the insertion between the vertebraes and operation tool for using the implant
Implant pour poser entre les vertèbres et outil d'operation pour l'utilisation de l'implant

(30) Priorität: 27.12.2000 DE 10065232
(43) Veröffentlichungstag der Anmeldung: 03.07.2002
(60) Teilanmeldung: 06016301.1
(73) Patentinhaber: Ulrich GmbH & Co. KG, 89081 Ulm (DE)
(72) Erfinder: Neumann, Carsten, Dr.med., 93007 Bad Abbach (DE)
(74) Vertreter: Hentrich, Swen

(56) Entgegenhaltungen:
- WO-A-00/23013
- DE-A- 3 023 942
- DE-A- 4 423 257
- DE-A- 19 622 827
- US-B1- 6 190 414

## Beschreibung

Implantat zum Einsetzen zwischen Wirbelkörper sowie Operationsinstrument zur Handhabung des Implantats gemäss dem Oberbegriff des Patentanspruchs 1.

Die Erfindung betrifft ein Implantat zum Einsetzen zwischen Wirbelkörper der Wirbelsäule als Platzhalter für aus der Wirbelsäule entfernte Wirbel oder Wirbelteile mit einem ersten Implantatteil und einem zweiten Implantatteil, die in Richtung ihrer koaxialen Längsachse zur Längenänderung des Implantats gegeneinander verstellbar sind. Die Erfindung betrifft weiterhin ein Operationsinstrument, mit dem die Längenänderung des Implantats bewirkt werden kann.

Ein Implantat der eingangs genannten Art ist beispielsweise aus der DE 44 23 257 A1 bekannt, das sich in der Praxis sehr gut bewährt hat und mit dem sehr gute Heilungserfolge nach der Implantation erzielt werden konnten. Während der Implantation bei der Operation an der offenen Wirbelsäule hat es sich allerdings als teilweise problematisch gezeigt, daß zum Verschrauben des ersten Implantatteils gegenüber dem zweiten Implantatteil ein großes Operationsfeld benötigt wird, da mittels Schwenkbewegungen die gegenseitige Verdrehung bewirkt werden muß. Platz für derartige Schwenkbewegungen steht aber nicht in jedem Fall ausreichend zur Verfügung, insbesondere wenn im Sinne einer minimal-invasiven Chirurgie nur kleine Zugänge zur Wirbelsäule gelegt werden.

Der Erfindung liegt daher die Aufgabe zugrunde, ein Implantat der eingangs genannten Art so auszubilden, daß eine Längenänderung des Implantats auch bewirkt werden kann, ohne daß dazu eine Schwenkbewegung des zur Betätigung des Implantats vorgesehenen Operationsinstrumentes erforderlich ist. Aufgabe der Erfindung ist weiterhin, ein Operationsinstrument bereitzustellen, das besonders gut zum Zusammenwirken mit dem erfindungsgemäßen Implantat geeignet ist.

Der das Implantat betreffende Teil der Aufgabe wird bei einem Implantat der eingangs genannten Art dadurch gelöst, daß dem ersten Implantatteil ein drehbarer Gewindering zugeordnet ist, der mit einem Ringgewinde in ein dem zweiten Implantatteil zugeordnetes Gewinde eingreift, und daß der Gewindering mit einer Kegelradverzahnung versehen ist.

Dieses Implantat bietet den großen Vorteil, daß aufgrund der Kegelradverzahnung eine Verdrehung des zweiten Implantatteils durch ein rein radial an das Implantat angesetztes Operationsinstrument bewirkt werden kann allein durch dessen Verdrehen. Damit ist zur Längenänderung ein im wesentlichen punktförmiger Zugang zum Implantat ausreichend, wodurch eine allein zum Zwecke der Distraktion vergrößerte Wunde vermieden ist.

Nach einer bevorzugten Ausführungsform der Erfindung ist vorgesehen, daß das erste Implantatteil und das zweite Implantatteil als rohrförmige Hülsen gestaltet sind, und daß das erste Implantatteil mit dem Gewindering das zweite Implantatteil außenseitig umfaßt. Diese Gestaltung der Erfindung unter Verwendung zweier Hülsen bietet den Vorteil, daß der Innenraum des Implantats frei ist und genutzt werden kann, um beispielsweise Knochenzement oder patienteneigene oder fremde Knochenspäne einzufüllen, um das Implantat zuverlässig einwachsen zu lassen. Um bei der Längenänderung des Implantats bei dem Verschrauben des zweiten Implantatteils gegenüber dem ersten Implantatteil eine Klemmung bzw. Selbsthemmung des Gewindes zu vermeiden, ist das Implantat so gestaltet, daß im zweiten Implantatteil mindestens ein Führungsschlitz zur Aufnahme eines dem ersten Implantatteil zugeordneten Stiftes ausgebildet ist. Zum Zwecke einer verbesserten Führung hat sich dabei bewährt, wenn der Führungsschlitz und der Stift zweifach in diametraler Anordnung vorgesehen sind.

Um die Betätigung des Gewinderinges mittels eines Operationsinstrumentes zu vereinfachen, ist in der Wandung des hülsenförmigen ersten Implantatteiles mindestens ein Durchbruch vorhanden, der den dem Gewindering zugeordneten Rand schneidet. Dieser Durchbruch kann als ein Lager für das an das Implantat anzusetzende Operationsinstrument genutzt werden.

Um die Führung des Operationsinstrumentes nicht auf die Dicke der Hülsenwand beschränken zu müssen, sind fluchtend mit dem Durchbruch im zweiten Implantatteil zwei Langlöcher vorhanden. Diese Langlöcher ermöglichen auch, daß gegenüberliegend zum Durchbruch in der Wandung des ersten Implantatteils eine Gewindebohrung ausgebildet ist, die von dem Durchbruch dann erreichbar ist.

Im Hinblick auf ein schnelleres Einwachsen des Implantats hat es sich weiterhin als günstig erwiesen, wenn am zweiten Implantatteil an dem dem ersten Implantatteil abgewandten Ende ein Kranz ausgebildet ist. Dieser Kranz vergrößert die Anlagefläche des zweiten Implantatteils an dem Wirbelkörper. Ist der Kranz mit Abstand vom freien Ende des zweiten Implantatteils angeordnet, so kann er als Sicherung für einzubringenden Knochenzement dienen und zugleich das Gewinde gegen das Zusetzen durch Knochenzement schützen. Alternativ ist es gleichfalls möglich, daß in dem Kranz parallel zur Längsachse des Implantats verlaufende Löcher ausgebildet sind.

Um eine möglichst große Führungslänge des Gewinderinges auf dem Gewinde zu erreichen, ist das Implantat so gestaltet, daß der Gewindering am Innenumfang einen Ringbund aufweist.

Um eine Sicherung der Lage des Gewinderinges gegenüber dem ersten Implantatteil bewirken zu können, ist das erfindungsgemäße Implantat weiterhin derartig gestaltet, daß das erste Implantatteil eine in der rohrförmigen Hülse ausgebildete, senkrecht zur Längsachse des Implantats verlaufende Quernut aufweist. Zweckmäßigerweise liegt diese Quernut parallel zur Verbindungsachse des Durchbruchs und der Gewindebohrung. Günstig ist weiterhin, wenn die Quernut beidseits der Verbindungsachse in der rohrförmigen Hülse des ersten Implantatteils ausgebildet ist.

Der das Operationsinstrument betreffende Teil der Aufgabe wird gelöst durch ein Operationsinstrument zur Distraktion eines über eine Gewindeverbindung längenverstellbaren, eine Kegelradverzahnung aufweisenden Implantats zum Einsetzen zwischen Wirbelkörper der Wirbelsäule als Platzhalter für aus der Wirbelsäule entfernte Wirbel, wobei an dem freien Ende einer Welle ein zur Wellenachse koaxiales Ritzel mit einer Kegelradverzahnung angeordnet ist. Durch dieses Operationsinstrument kann durch die radial von dem Implantat wegstehende Welle, die senkrecht zur Längsachse des Implantats orientiert ist, allein durch Verdrehung um die Längsachse der Welle die Kegelradverzahnung des Ritzels verdreht werden, das bei dem Zusammenwirken mit der Kegelradverzahnung des Gewinderinges die zur Längenänderung des Implantats erforderliche Drehung des ersten Implantatteils und des zweiten Implantatteils relativ zueinander bewirkt.

Zur leichteren Handhabung des Operationsinstruments während der Operation ist vorgesehen, daß die Welle drehbar in einem Handgriff gelagert und zum Antrieb mit einem Rändelrad an dem dem Ritzel abgewandten Ende versehen ist.

Eine besonders bevorzugte Ausführungsform des Operationsinstruments ist dadurch gekennzeichnet, daß die Welle als Hohlwelle gestaltet ist, in der ein gegenüber der Hohlwelle verdrehbarer Stab gelagert ist, der das Ritzel mit seinem freien Ende durchgreift und an dem ein zu der Gewindebohrung korrespondierendes Stabgewinde ausgebildet ist. Dies bietet den Vorteil, daß die Lage des ersten Implantatteils gegenüber dem Operationsinstrument durch das Stabgewinde gesichert werden kann, so daß durch die Ausübung des notwendigen Druckes, um die Kegelradverzahnung des Ritzels in Eingriff mit der Kegelradverzahnung des Gewinderinges zu bringen, eine Lageänderung des bereits zwischen den Wirbelkörpern der Wirbelsäule plazierten Platzhalters nicht provoziert wird.

Eine weitere bevorzugte Ausführungsform der Erfindung ist dadurch gekennzeichnet, daß die Welle in einer an dem Handgriff befestigten Hülse angeordnet ist, die an dem dem Ritzel zugeordneten Ende eine Gabel mit einer Sicherung der gegenseitigen axialen Lage des Gewinderinges und des ersten Implantatteils bewirkenden Gabelfingern aufweist. Als günstig hat sich dabei erwiesen, wenn die Gabel insgesamt vier das Implantat haltende Gabelfinger aufweist, von denen zwei zu einem ersten Gabelpaar zusammengefaßt und zur Anlage auf dem Gewindering und zwei zu einem zweiten Gabelpaar zusammengefaßt und zum Einführen in die Quernuten vorgesehen sind. Mit einem derartigen Operationsinstrument kann das Implantat sehr sicher erfaßt und an einem vorbestimmten Ort gehalten werden, wobei trotz der erforderlichen Verdrehung der Welle zur Längenänderung keine Gefahr besteht, daß das Implantat im Operationsfeld verrutscht oder das erste Implantat und das zweite Implantat sich gegeneinander verschieben und so eine Längenerstreckung einnehmen, die nicht durch eine Verdrehung des Gewinderinges bewirkt wird und durch diesen abgesichert ist.

Im Hinblick auf verbesserte hygienische Verhältnisse bei der Desinfektion ist es zweckmäßig, wenn die Welle mit dem Rändelrad, der Stab und der Handgriff mit der Hülse zerlegbar montiert sind.

Im folgenden soll die Erfindung an einem in der Zeichnung dargestellten Ausführungsbeispiel näher erläutert werden; es zeigen:
- Fig. 1: eine perspektivische Darstellung des erfindungsgemäßen Implantates bei der Betätigung durch das erfindungsgemäße Operationsinstrument,
- Fig. 2: eine perspektivische Darstellung des Implantats isoliert,
- Fig. 3: eine perspektivische Darstellung des zweiten Implantatteils,

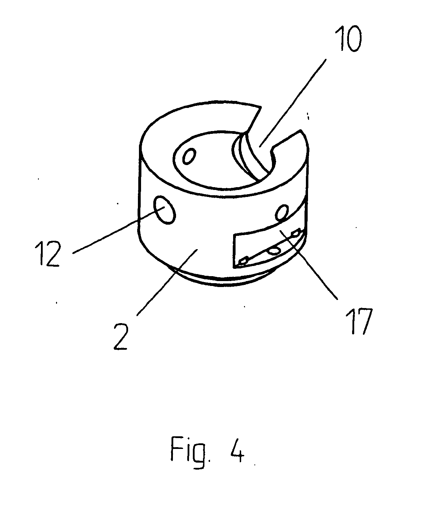

- Fig. 4: eine perspektivische Darstellung des ersten Implantatteils,

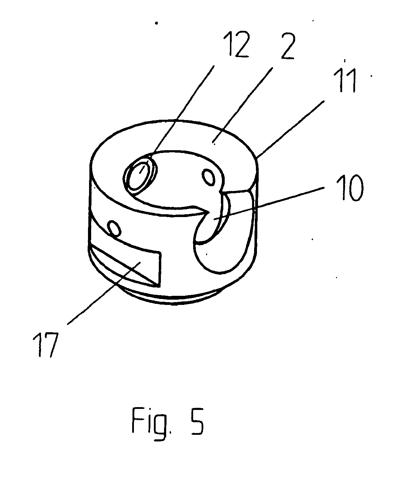

- Fig. 5: eine perspektivische Darstellung des ersten Implantatteils aus einer gegenüber Fig. 4 anderen Sicht,
- Fig. 6: eine Seitenansicht des zweiten Implantatteils,

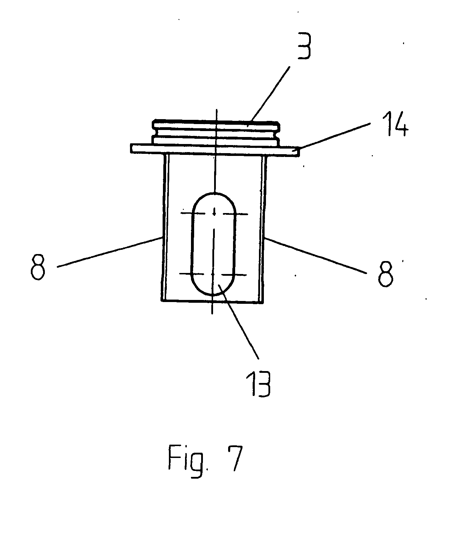

- Fig. 7: eine Seitenansicht des zweiten Implantatteils mit gegenüber Fig. 6 um 90° gedrehter Darstellung,
- Fig. 8: einen Querschnitt durch den die Kegelradverzahnung aufweisenden Gewindering,
- Fig. 9: das in seine Einzelteile zerlegte, erfindungsgemäße Operationsinstrument in einer Draufsicht,
- Fig. 10: eine perspektivische Darstellung des Operationsinstrumentes komplett montiert und in seine Einzelteile zerlegt,

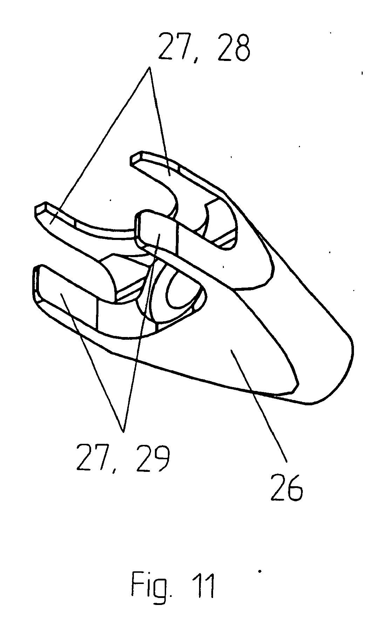

- Fig. 11: eine perspektivische Darstellung der Gabel der Hülse und

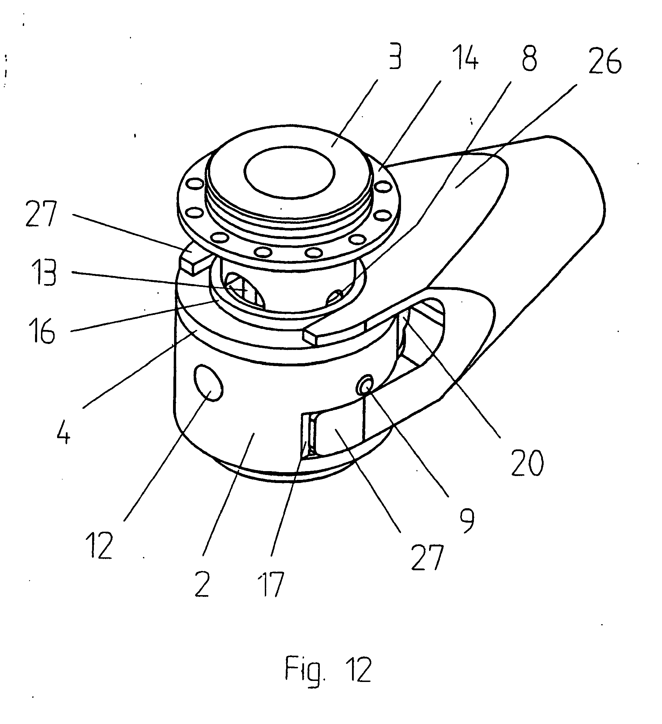

- Fig. 12: die Detaildarstellung XII aus Fig. 1.

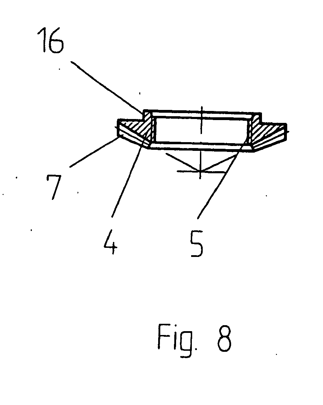

Das in der Zeichnung dargestellte Implantat 1 dient zum Einsetzen zwischen in der Zeichnung selber nicht dargestellte Wirbelkörper der Wirbelsäule als Platzhalter für aus der Wirbelsäule entfernte Wirbel oder Wirbelteile. Dieses Implantat 1 weist ein erstes Implantatteil 2 und ein zweites Implantatteil 3 auf, die in Richtung ihrer koaxialen Längsachse gegeneinander verstellbar sind, um im Sinne einer Distraktion der Wirbelkörper eine Längenänderung bewirken zu können. Das erste Implantatteil 2 und das zweite Implantatteil 3 sind als rohrförmige Hülsen gestaltet, wobei das erste Implantatteil 2 das zweite Implantatteil 3 außenseitig umfaßt. Dem ersten Implantatteil 2 ist ein drehbarer Gewindering 4 zugeordnet, der an seiner Innenumfangsfläche ein Ringgewinde 5 aufweist, mit dem dieser in ein dem zweiten Implantatteil 3 zugeordnetes Gewinde 6 eingreift. An der Außenumfangsfläche ist der Gewindering 5 mit einer Kegelradverzahnung 7 versehen (Fig. 8).

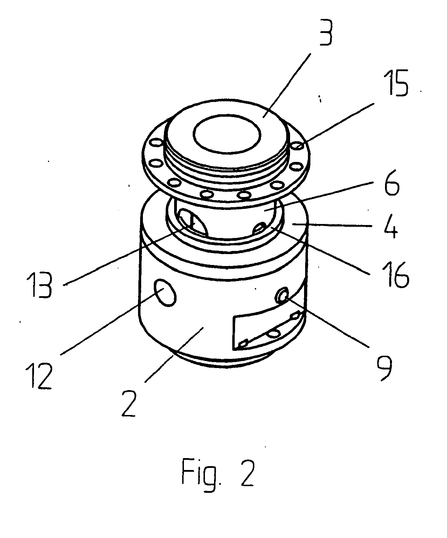

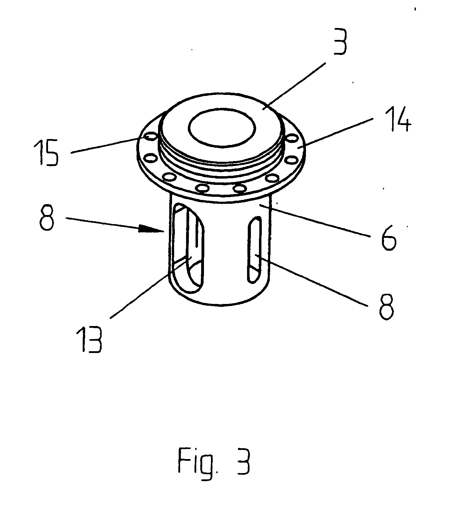

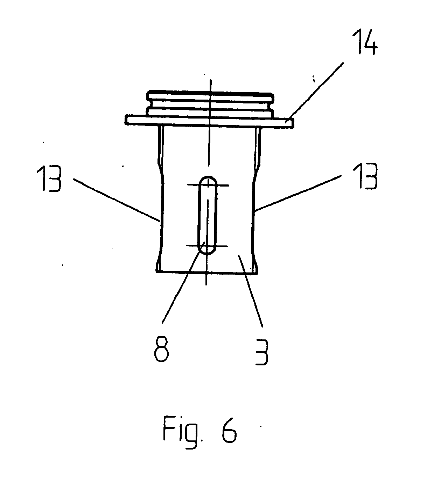

Wie insbesondere aus den Figuren 3 und 6 zu erkennen ist, sind im zweiten Implantatteil 3 zwei einander gegenüberliegende Führungsschlitze 8 ausgebildet, in die dem ersten Implantatteil 2 zugeordnete Stifte 9 hineinragen (Fig. 2).

Neben diesen Stiften 9 weist das erste Implantatteil 2 weiterhin einen Durchbruch 10 auf, der den dem Gewindering 4 zugeordneten Rand 11 schneidet und auf einer Geraden mit einer auf der gegenüberliegenden Seite ausgebildeten Gewindebohrung 12 liegt. Fluchtend sind mit dem Durchbruch 10 in dem zweiten Implantatteil 3 zwei Langlöcher 13 vorgesehen, durch die hindurch die Gewindebohrung 12 in jedweder Höhenlage des zweiten Implantatteils 3 gegenüber dem ersten Implantatteil 1 erreichbar ist.

An dem zweiten Implantatteil 3 ist mit Abstand von dessen freien Ende auf der dem ersten Implantatteil 2 abgewandten Seite ein Kranz 14 ausgebildet, in dem parallel zur Längsachse des Implantats 1 verlaufende Löcher 15 ausgebildet sind.

Der Gewindering 4 weist am Innenumfang einen Ringbund 16 auf, während das erste Implantatteil 2 eine in der rohrförmigen Hülse ausgebildete, senkrecht zur Längsachse des Implantats 1 verlaufende Quernut 17 aufweist, die parallel zur Verbindungsachse des Durchbruchs 10 und der Gewindebohrung 12 liegt, wobei bei dem in der Zeichnung dargestellten Ausführungsbeispiel die Quernut 17 zweifach beidseits der Verbindungsachse vorgesehen ist.

Der am Gewindering 4 ausgebildete Ringbund 16 sowie die Quernuten 17 dienen zur besseren Ausrichtung und Bestimmung der Lage eines nachfolgend näher zu beschreibenden Operationsinstrumentes 18, das zur Handhabung und Betätigung des Implantats 1 bei der Längenverstellung dient.

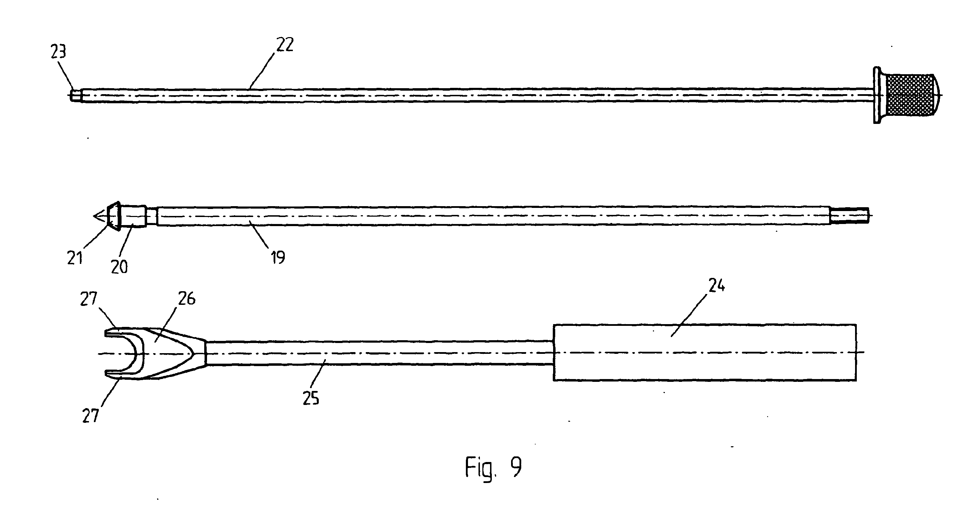

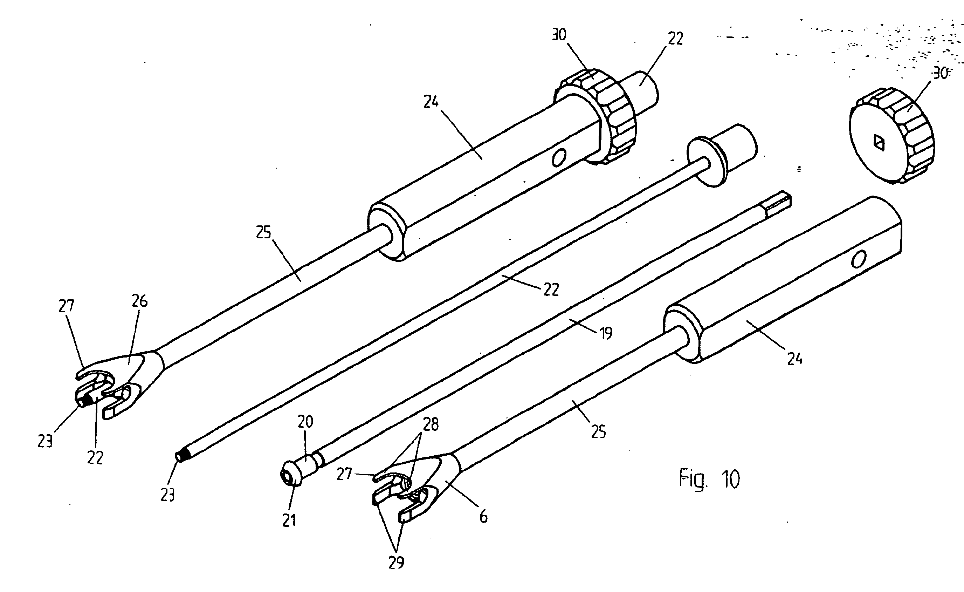

Das Operationsinstrument 18 weist dazu an dem freien Ende einer Welle 19 ein zur Wellenachse koaxiales Ritzel 20 auf. An diesem Ritzel 20 ist eine Kegelradverzahnung 21 ausgebildet (Fig. 9, Fig. 10). Die Welle 19 selber ist als Hohlwelle gestaltet, in der ein Stab 22 gegenüber der Hohlwelle verdrehbar gelagert ist. Dieser Stab 22 durchgreift das Ritzel 20 und weist an seinem freien Ende ein zu der Gewindebohrung 12 korrespondierendes Stabgewinde 23 auf. Die Hohlwelle selber ist drehbar in einem Handgriff 24 gelagert, der dazu eine Hülse 25 aufweist, in die die Hohlwelle eingeführt ist. Diese Hülse 25 besitzt an dem dem Ritzel 20 zugeordneten Ende eine Gabel 26 mit vier Gabelfingern 27, von denen zwei zu einem ersten Gabelpaar 28 zusammengefaßt und zwei zu einem zweiten Gabelpaar 29 zusammengefaßt sind.

Nachdem vorstehend der Aufbau des Implantats 1 und des Operationsinstrumentes 18 geschildert ist, soll nachfolgend deren Zusammenwirken detaillierter dargelegt werden.

Zum Plazieren des erfindungsgemäßen Implantats 1 als Platzhalter zwischen zwei Wirbelkörpern wird zunächst das Implantat 1 auf die Gabel 26 gesteckt, wobei das zweite Gabelpaar 29 in die beiden Quernuten 17 eingeführt wird und das erste Gabelpaar 28 auf dem Gewindering 4 und radial innen an dem Ringbund 16 zur Anlage kommt, so daß eine axiale Verschiebung des Gewinderinges 4 in der Längsachse des Implantats 1 gegenüber dem ersten Implantatteil 2 blockiert ist. Anschließend wird der Stab 22 mit dem Stabgewinde 23 durch den Durchbruch 10 des ersten Implantatteils 2 sowie die beiden Langlöcher 13 des zweiten Implantatteils 3 geführt und in die Gewindebohrung 12 des ersten Implantatteils 2 eingeschraubt, so daß das Operationsinstrument 18 nicht mehr in radialer Richtung von dem Implantat 1 abgezogen werden kann, die Lage des Operationsinstrumentes 18 damit insgesamt gegenüber dem Implantat 1 gesichert ist. Durch das Einschrauben des Stabgewindes 23 in die Gewindebohrung 12 wird zugleich die Kegelradverzahnung 21 des Ritzels 20 in Eingriff mit der Kegelradverzahnung 7 des Gewinderinges 4 gebracht, so daß bei der nachfolgenden Verdrehung der Welle 19 um ihre Wellenachse durch ein Rändelrad 30 eine Verdrehung des Gewinderinges 4 um die zur Wellenachse um 90° gedrehte Längsachse des Implantats 1 erfolgt.

Nach der korrekten Plazierung und Distraktion des Implantats 1 kann das Operationsinstrument 18 in sinngemäßer Umkehrung der vorstehend beschriebenen Schritte wieder von dem Implantat 1 gelöst werden, das durch die dann frei zugänglichen Führungsschlitze 8, Langlöcher 13 und die Gewindebohrungen 12 sowie den Durchbruch 10 genügend Zugangsmöglichkeiten in das Innere der hülsenförmigen ersten Implantatteile 2 und zweiten Implantatteile 3 bietet, um Knochenzement oder autologes oder homologes Knochenmaterial in das Implantat einzubringen.

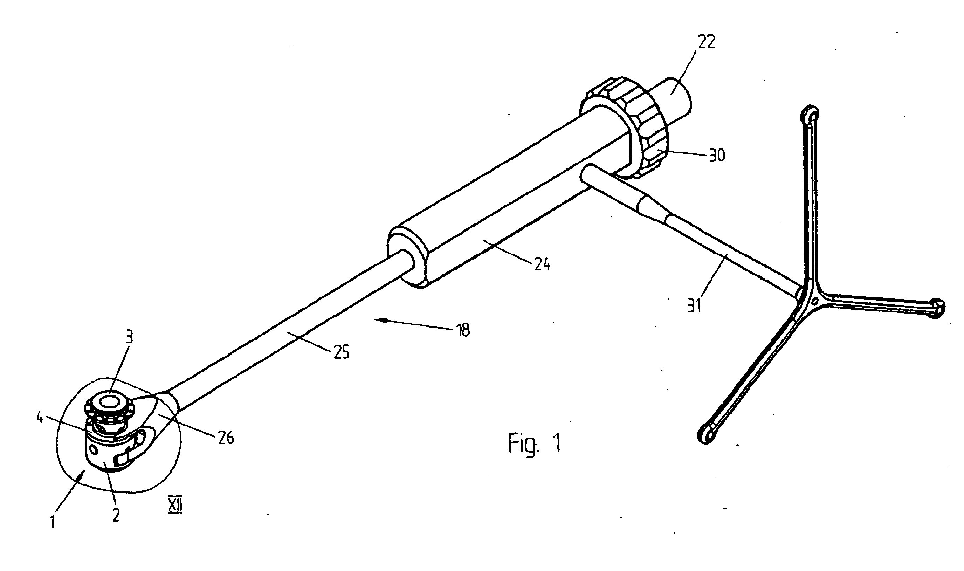

Der in Figur 1 dargestellte mit dem Bezugszeichen 31 bezeichnete Aufsatz dient der Kopplung des Operationsinstruments 18 mit Positionsgebern für die Computer-Navigation.

## Patentansprüche

1. Implantat zum Einsetzen zwischen Wirbelkörper der Wirbelsäule als Platzhalter für aus der Wirbelsäule entfernte Wirbel oder Wirbelteile, mit einem ersten Implantatteil (2) und einem zweiten Implantatteil (3), die in Richtung ihrer koaxialen Längsachse zur Längenänderung des Implantats (1) gegeneinander verstellbar sind, wobei dem ersten Implantatteil (2) ein drehbarer, mit einer Kegelradverzahnung (7) versehener Gewindering (4) zugeordnet ist, der mit einem Ringgewinde (5) in ein dem zweiten Implantatteil (3) zugeordnetes Gewinde (6) eingreift, und wobei in der Wandung des ersten Implantatteils (2) eine Gewindebohrung (12) ausgebildet ist, **dadurch gekennzeichnet, dass**, dass das erste Implantatteil (2) und das zweite Implantatteil (3) als rohrförmige Hülsen gestaltet sind und das erste Implantatteil (2) mit dem Gewindering (4) das zweite Implantatteil (3) aussenseitig umfasst, dass im zweiten Implantatteil (3) mindestens ein Führungsschlitz (8) zur Aufnahme eines dem ersten Implantatteil (2) zugeordneten Stiftes (9) ausgebildet ist, dass in der Wandung des hülsenförmigen ersten Implantatteils (2) mindestens ein Durchbruch (10) vorhanden ist, der den dem Gewindering (4) zugeordneten Rand (11) schneidet, dass fluchtend mit dem Durchbruch (10) im zweiten Implantatteil (3) zwei Langlöcher (13) vorhanden sind, und, dass gegenüberliegend zum Durchbruch (10) in der Wandung des ersten Implantatteils (2) die Gewindebohrung (12) ausgebildet ist.

2. Implantat nach Anspruch 1, **dadurch gekennzeichnet, dass** der Führungsschlitz (8) und der Stift (9) zweifach in diametraler Anordnung vorgesehen sind.

3. Implantat nach einem der Ansprüche 1 bis 2, **dadurch gekennzeichnet, dass** am zweiten Implantatteil (3) an dem dem ersten Implantatteil (2) abgewandten Ende ein Kranz (14) ausgebildet ist.

4. Implantat nach Anspruch 3, **dadurch gekennzeichnet, dass** der Kranz (14) mit Abstand vom freien Ende des zweiten Implantatteils (3) angeordnet ist.

5. Implantat nach Anspruch 3 oder 4, **dadurch gekennzeichnet, dass** in dem Kranz (14) parallel zur Längsachse des Implantats (1) verlaufende Löcher (15) ausgebildet sind.

6. Implantat nach einem der Ansprüche 1 bis 5, **dadurch gekennzeichnet, dass** der Gewindering (4) am Innenumfang einen Ringbund (16) aufweist.

7. Implantat nach einem der Ansprüche 1 bis 6, **dadurch gekennzeichnet, dass** das erste Implantatteil (2) eine in der rohrförmigen Hülse ausgebildete, senkrecht zur Längsachse des Implantats (1) verlaufende Quernut (17) aufweist.

8. Implantat nach Anspruch 7, **dadurch gekennzeichnet, dass** die Quernut (17) parallel zur Verbindungsachse des Durchbruchs (10) und der Gewindebohrung (12) liegt.

9. Implantat nach Anspruch 8, **dadurch gekennzeichnet, dass** die Quernut (17) beidseits der Verbindungsachse in der rohrförmigen Hülse des ersten Implantatteils (2) ausgebildet ist.

10. Operationsinstrument zur Distraktion eines über eine Gewindeverbindung längenverstellbaren, eine Kegelradverzahnung (7) aufweisenden Implantats (1) zum Einsetzen zwischen Wirbelkörper der Wirbelsäule als Platzhalter für aus der Wirbelsäule entfernte Wirbel oder Wirbelteile, nach einem der Ansprüche 1 bis 9, wobei an dem freien Ende einer Welle (19) ein zur Wellenachse koaxiales Ritzel (20) mit einer Kegelradverzahnung (21) angeordnet ist, **dadurch gekennzeichnet, dass** die Welle (19) als Hohlwelle gestaltet ist, in der ein gegenüber der Hohlwelle verdrehbarer Stab (22) gelagert ist, der das Ritzel (20) mit seinem freien Ende durchgreift und an dem ein zu der Gewindebohrung (12) korrespondierendes Stabgewinde (23) ausgebildet ist.

11. Operationsinstrument nach Anspruch 10, **dadurch gekennzeichnet, dass** die Welle (19) drehbar in einem Handgriff (24) gelagert und zum Antrieb mit einem Rändelrad (30) an dem dem Ritzel (20) abgewandten Ende versehen ist.

12. Operationsinstrument nach Anspruch 10 oder 11, **dadurch gekennzeichnet, dass** die Welle (19) in einer an dem Handgriff (24) befestigten Hülse (25) angeordnet ist, die an dem dem Ritzel (20) zugeordneten Ende eine Gabel (26) mit eine Sicherung der gegenseitigen axialen Lage des Gewinderinges (4) und des ersten Implantatteils (2) bewirkenden Gabelfingern (27) aufweist.

13. Operationsinstrument nach Anspruch 12, **dadurch gekennzeichnet, dass** die Gabel (26) insgesamt vier das Implantat (1) haltende Gabelfinger (27) aufweist, von denen zwei zu einem ersten Gabelpaar (28) zusammengefasst und zur Anlage auf den Gewindering (4) und zwei zu einem zweiten Gabelpaar (29) zusammengefasst und zum Einführen in die Quernuten (17) vorgesehen sind.

14. Operationsinstrument nach einem der Ansprüche 10 bis 13, **dadurch gekennzeichnet, dass** die Welle (19) mit dem Rändelrad (30), der Stab (22) und der Handgriff (24) mit der Hülse (25) zerlegbar montiert sind.

## Claims

1. An implant for insertion between vertebrae of the spinal column as a place holder for vertebrae or spinal portions which have been removed from the spinal column, comprising a first implant portion (2) and a second implant portion (3) which are displaceable relative to each other in the direction of their coaxial longitudinal axis for altering the length of the implant (1), wherein associated with the first implant portion (2) is a rotatable screwthreaded ring (4) which is provided with a bevel gear tooth array (7) and engages with a ring screwthread (5) into a screwthread (6) associated with the second implant portion (3), and wherein a screwthreaded bore (12) is provided in the wall of the first implant portion (2), **characterised in that** the first implant portion (2) and the second implant portion (3) are in the form of tubular sleeves and the first implant portion (2) with the screwthreaded ring (4) externally embraces the second implant portion (3), that provided in the second implant portion (3) is at least one guide slot (8) for receiving a pin (9) associated with the first implant portion (2), that provided in the wall of the sleeve-shaped first implant portion (2) is at least one aperture (10) which intersects the edge (11) associated with the screwthreaded ring (4), that two elongate holes (13) are provided in alignment with the aperture (10) in the second implant portion (3), and that the screwthreaded bore (12) is provided in opposite relationship to the aperture (10) in the wall of the first implant portion (2).

2. An implant according to claim 1 **characterised in that** the guide slot (8) and the pin (9) are provided in duplicate in a diametral arrangement.

3. An implant according to one of claims 1 and 2 **characterised in that** a rim (14) is provided on the second implant portion (13) at the end remote from the first implant portion (2).

4. An implant according to claim 3 **characterised in that** the rim (14) is arranged at a spacing from the free end of the second implant portion (3).

5. An implant according to claim 3 or claim 4 **characterised in that** holes (15) extending in parallel relationship with the longitudinal axis of the implant (1) are provided in the rim (14).

6. An implant according to one of claims 1 to 5 **characterised in that** the screwthreaded ring (4) has at the inside periphery an annular collar (16).

7. An implant according to one of claims 1 to 6 **characterised in that** the first implant portion (2) has a transverse groove (17) which is provided in the tubular sleeve and which extends perpendicularly to the longitudinal axis of the implant (1).

8. An implant according to claim 7 **characterised in that** the transverse groove (17) is parallel to the line connecting the aperture (10) and the screwthreaded bore (12).

9. An implant according to claim 8 **characterised in that** the transverse groove (17) is provided on both sides of the connecting line in the tubular sleeve of the first implant portion (2).

10. An operating instrument for the distraction of an implant (1) which is adjustable in length by way of a screwthread connection and which has a bevel gear tooth array (7) for insertion between vertebrae of the spinal column as a place holder for vertebrae or vertebrae portions removed from the spinal column, according to one of claims 1 to 9, wherein arranged at the free end of a shaft (19) is a pinion (20) which is coaxial with the axis of the shaft and which has a bevel gear tooth array (21), **characterised in that** the shaft (19) is in the form of a hollow shaft in which there is supported a rod (22) which is rotatable with respect to the shaft and which engages through the pinion (20) with its free end and on which there is a rod screwthread (23) corresponding to the screwthreaded bore (12).

11. An operating instrument according to claim 10 **characterised in that** the shaft (19) is supported rotatably in a handle (24) and for drive purposes is provided with a knurled wheel (30) at the end remote from the pinion (20).

12. An operating instrument according to claim 10 or claim 11 **characterised in that** the shaft (19) is arranged in a sleeve (25) which is fixed to the handle (24) and which at the end associated with the pinion (20) has a fork (26) with fork fingers (27) effecting securing of the mutual axial position of the screwthreaded ring (4) and the first implant portion (2).

13. An operating instrument according to claim 12 **characterised in that** the fork (26) in total has four fork fingers (27) which hold the implant (1) and of which two are combined to afford a first fork pair (28) for bearing against the screwthreaded ring (4) and two are combined to afford a second fork pair (29) for insertion into the transverse groove (17).

14. An operating instrument according to one of claims 10 to 13 **characterised in that** the shaft (19) with the knurled wheel (30), the rod (22) and the handle (24) with the sleeve (25) are dismantleably assembled.

## Revendications

1. Implant à poser entre les vertèbres de la colonne vertébrale en tant que substitut d'une vertèbre ou de parties de vertèbre retirées de la colonne vertébrale, qui est constitué d'une première partie d'implant (2) et d'une seconde partie d'implant (3) qui peuvent être déplacées l'une par rapport à l'autre selon la direction de l'axe longitudinal commun, de manière à faire varier la longueur de l'implant (1), une bague filetée (4) rotative pourvue d'une denture de pignon conique (7) qui est en prise avec un filetage annulaire (5) réalisé dans un filetage (6) associé à la seconde partie d'implant, la bague annulaire (4) étant associée à la première partie d'implant (2) et un alésage fileté (12) étant aménagé dans la paroi de la première partie d'implant (2), **caractérisé en ce que** la première partie d'implant (2) et la seconde partie d'implant (3) sont des douilles de forme tubulaire, la première partie d'implant (2) entourant extérieurement par la bague filetée (4) la seconde partie d'implant (3), **en ce qu'**au moins une fente de guidage (8) pour accueillir une broche (9) associée à la première partie d'implant (2) est aménagée dans la seconde partie d'implant (3), **en ce que** dans la paroi de la première partie d'implant (2) en forme de douille, se trouve au moins un passage (10) qui coupe le bord (11) associé à la bague filetée (4), **en ce que** dans la seconde partie d'implant (3) se trouvent deux trous allongés (13) alignés avec le passage (10) et **en ce que** dans la paroi de la première partie d'implant se trouve un alésage fileté (12) disposé en face du passage (10).

2. Implant selon la revendication 1, **caractérisé en ce que** la fente de guidage (8) et la broche (9) sont doubles en étant diamétralement opposées.

3. Implant selon une des revendications 1 à 2, **caractérisé en ce qu'**une couronne (14) est réalisée sur la seconde partie d'implant (3) vers l'extrémité de celle-ci éloignée de la première partie d'implant (2).

4. Implant selon la revendication 3, **caractérisé en ce que** la couronne (14) est disposée à une certaine distance de l'extrémité libre de la seconde partie d'implant (3).

5. Implant selon la revendication 3 ou 4, **caractérisé en ce que** des trous (15) sont réalisés parallèlement à l'axe longitudinal de l'implant (1), dans la couronne (14).

6. Implant selon une des revendications 1 à 5, **caractérisé en ce que** la bague filetée (4) présente sur sa périphérie externe un collet annulaire (16).

7. Implant selon une des revendications 1 à 6, **caractérisé en ce que** la première partie d'implant (2) présente une rainure transversale (17) réalisée dans la douille tubulaire, perpendiculairement à l'axe longitudinal de l'implant (1).

8. Implant selon la revendication 7, **caractérisé en ce que** la rainure transversale (17) est parallèle à l'axe commun du passage (10) et de l'alésage fileté (12).

9. Implant selon la revendication 8, **caractérisé en ce que** la rainure transversale (17) dans la douille tubulaire de la première partie d'implant (2) est aménagée de part et d'autre de l'axe commun

10. Instrument opératoire pour étirer un implant (1) réglable en longueur par une liaison filetée et présentant une denture de pignon conique (17), afin de l'introduire entre la vertèbre de la colonne vertébrale en tant que substitut remplaçant une vertèbre ou des parties de vertèbre retirées de la colonne vertébrale selon une des revendications 1 à 9, un pignon coaxial (20) portant une denture de pignon conique (21) étant monté à l'extrémité libre d'un arbre (19), **caractérisé en ce que** l'arbre (19) est conformé en arbre creux à l'intérieur duquel une tige (22), dont l'extrémité libre pénètre dans le pignon (20) et sur laquelle il est prévu un filetage (23) associé à l'alésage fileté (12), est montée tournante par rapport audit arbre creux.

11. Instrument opératoire selon la revendication 10, **caractérisé en ce que** l'arbre (19) peut tourner dans une poignée (24) et porte à son extrémité éloignée du pignon (20) une bague moletée (30) pour assurer son entraînement.

12. Instrument opératoire selon la revendication 10 ou 11, **caractérisé en ce que** l'arbre (19) est monté dans une douille (25) qui est fixée à la poignée (24) et qui, à son extrémité associée au pignon (20), présente une fourche (26) avec des doigts (27) qui sécurisent la position axiale réciproque de la bague filetée (4) et de la première partie d'implant (2).

13. Instrument opératoire selon la revendication 12, **caractérisé en ce que** la fourche (26) comprend en tout quatre doigts de fourche (27) qui maintiennent l'implant (1), et dont deux constituent une première paire prévue pour venir en appui sur la bague filetée (4), tandis que les deux autres doigts forment une seconde paire (29) à engager dans les rainures transversales (17).

14. Instrument opératoire selon une des revendications 10 à 13, **caractérisé en ce que** l'arbre (19) avec la bague moletée (30), la tige (22) et la poignée (24), peuvent être montés de manière amovible sur la douille (25).
